# EUROPÄISCHE PATENTANMELDUNG

(11) **EP 2 979 714 A1**
(43) Veröffentlichungstag der Anmeldung: **03.02.2016**
(21) Anmeldenummer: 14179613.6
(22) Anmeldetag: 02.08.2014
(51) Int. Cl.: A61M 5/30

(54) **Nadellose Injektionsvorrichtung aufweisend eine Membran**

(71) Anmelder: LTS LOHMANN Therapie-Systeme AG, 56626 Andernach (DE)
(72) Erfinder: LELL, Peter, 85368 Moosburg (DE)
(74) Vertreter: Simandi, Claus

(57) **Zusammenfassung**

Die Erfindung betrifft eine nadellose Injektionsvorrichtung, enthaltend mindestens eine Membran, bzw. eine entsprechende Vorrichtung zur nadellosen Injektion einer Substanz aufweisend mindestens eine Membran mit der ein pulverförmiger, gelartiger oder flüssiger Stoff, insbesondere ein Wirkstoff, mittels hoher Auftreffgeschwindigkeit in ein Gewebe oder einen Körper nadellos injiziert werden kann. Weiterhin betrifft die Erfindung ein Verfahren zu einer solchen nadellosen Injektionsvorrichtung enthaltend eine Membran und dessen Verwendung.

## Beschreibung

Die Erfindung betrifft eine nadellose Injektionsvorrichtung, enthaltend mindestens eine Membran, bzw. eine entsprechende Vorrichtung zur nadellosen Injektion einer Substanz aufweisend mindestens eine Membran mit der ein pulverförmiger, gelartiger oder flüssiger Stoff, insbesondere ein Wirkstoff, mittels hoher Auftreffgeschwindigkeit in ein Gewebe oder einen Körper nadellos injiziert werden kann. Weiterhin betrifft die Erfindung ein Verfahren zu einer solchen nadellosen Injektionsvorrichtung enthaltend eine Membran und dessen Verwendung.

Eine erfolgreiche Injektion besteht darin, dass genügend Substanz durch die Gewebe-, oder Körperbarriere, insbesondere solche wie die Haut von Menschen oder Tieren, in den Körper eingebracht wird. Das Grundprinzip, einen Stoff nadellos mittels hohen Drucks zu injizieren, ist seit geraumer Zeit bekannt (siehe z. B. US 3,308,818).

Es sind verschiedene Typen von nadellosen Injektionsvorrichtungen im Stand der Technik beschrieben, wie z. B. nicht abschließend eine Zylinder-Kolben-Einheit umfassend ein vorgespanntes Federelement (DE 10 2007 004 211 A1) oder mittels sogenannter Injektionspatronen (WO 98/31409) als auch mittels einer Gaspatrone zur Beaufschlagung eines Kolbens (US 5,399,163).

Weiterhin können nadellose Injektionsvorrichtungen aus einem pyrotechnischen Antrieb bestehen, der beispielsweise zur Betätigung eines Kolbens genutzt wird und auf diese Weise das Auspressen eines Wirkstoffes aus einer Kanüle erzeugt (EP 1 557 190 A1).

Eine weitere Ausführungsform einer nadellosen Injektionsvorrichtung besteht in einem pyrotechnischen Ansatz, wobei in einer Kammer (auch Brennkammer genannt) eine Explosion erzeugt wird, wobei die freigesetzte Energie zur Impulsübertragung auf eine Membran genutzt wird, wodurch eine membranständige adsorbierte Substanz unter Ablösung von der Membran in Richtung des Gewebes hinreichend hoch beschleunigt wird.

Eine solche Vorrichtung ist zum Beispiel in WO 2004/071558 A1 offenbart. Es gilt jedoch eine solche gattungsmäßige Ausführungsform zu verbessern, so dass eine verbesserte Einbringung einer gewünschten Substanz in einen Körper erfolgen kann.

Von daher nimmt die vorliegende Erfindung Bezug auf eine gattungsmäßige Ausführungsform gemäß WO 2004/071558, jedoch mit der objektiven Aufgabenstellung, die nadelfreie Injektion zu verbessern.

Daher betrifft die vorliegende Erfindung eine nadellose Injektionsvorrichtung umfassend eine hautseitige Membran (15), die auch wie nachfolgend beschrieben als Doppel- oder Mehrfachmembran ausgebildet sein kann, wobei die Membran (15) mit einer Partikelauftragung (25) versehen ist, wobei mindestens ein Teil der Partikel größer ist als die restlichen Partikel, weiterhin ist bevorzugt, dass mindestens ein Teil der Partikel mindestens 2-fach, 5-fach, 10-fach oder 20-fach größer ist als die restlichen Partikel.

In einer weiteren Ausführungsform ist bevorzugt, dass mindestens ein oder mehrere größere Partikel in der Gesamtheit der Partikelauftragung (25) neben kleineren Partikeln enthalten sind. Weiterhin kann der Anteil der größeren Partikel 1%, 5%, 10 % und mehr an der Gesamtheit der Partikelauftragung betragen.

Im Rahmen dieser Erfindung wird unter "Partikel" z.B. Staub, Körner, Kristalle, Pulver oder kleine feste Teilchen, z.B. nicht abschließend Zucker, Glas-, Kunststoff oder Metallteilchen, jedoch auch Gel- und Flüssigkeitsteilchen verstanden, die eine Größe von ca. 1 nm -1.000 µm aufweisen können. Diese Gel- oder Flüssigkeitsteilchen können aus einem Gel- oder Flüssigkeitsfilm auf der Membran durch dessen stoßartiger Beschleunigung und Abbremsung bei der Ablösung des Gels bzw. der Flüssigkeit entstehen.

Beispielsweise können solche Partikel aus pharmazeutisch verträglichen oder unbedenklichen Materialien, solche die leicht vom Körper abbaubar sind, wie z.B. Zucker (Glucose etc.) bestehen. Wesentlich ist, dass eine Partikelauftragung (25) besteht, d.h. eine Membran (15) wird mit Partikeln bestäubt oder mit einer Gel- oder Flüssigkeitsschicht beschichtet. Solche Techniken sind dem Fachmann einschlägig bekannt. Weiterhin können die Partikel ebenfalls aus einer Reinsubstanz bestehen oder ein Substanzgemisch darstellen. Beispielsweise können die Partikel einen Wirkstoff enthalten, oder vollständig aus einem Wirkstoff bestehen.

Erfindungswesentlich ist nunmehr, dass ein oder mehrere größere Partikel neben kleineren Partikeln in der Partikelauftragung vorliegen, wobei es die Aufgabe von ein oder mehreren größeren Partikel ist, die Haut bzw. den Körper unter Öffnung zu penetrieren, wie gewünscht tief einzudringen und auf diese Weise vorteilhaft für die nachfolgenden kleineren Partikel eine entsprechend tief in die Haut oder den Körper eindringende Wirkfläche zur Bestäubung zu schaffen und im Ergebnis das Penetrieren und Eindringen der kleineren Partikel vorteilhaft zu erleichtern.

Ohne die Verwendung von mindestens einem oder mehrerer größerer Partikel können die kleineren Partikel bei der durch das Applikationsgerät / Membrangerät verfügbaren Auftreffgeschwindigkeit nicht ausreichend tief in das Hautgewebe bzw. in den Körper eindringen. Die gewünschte Eindringtiefe wird vorteilhaft durch die Eigenschaften der größeren Partikel eingestellt. Maßgebend sind die vom Membrangerät vorgegebene Austrittsgeschwindigkeit, die Größe als auch die Masse der besagten größeren Partikel, die quasi als Öffner dienen und eben mit der dritten Potenz des Radius und der spezifischen Dichte des Stoffes ansteigt als auch die Geometrie der besagten größeren Partikel im Augenblick des Auftreffens auf der Haut oder der Körperoberfläche.

Daher werden vorteilhaft und in überraschender Weise durch die größeren Partikel schlauchund kapillarartige Öffnungen als auch Eindringkegel (bzw. Überschallkegel) in der Haut erhalten, so dass zum einen die Oberfläche vergrößert wird und zum anderen Kapillarkräfte genutzt werden können. Diese schlauch- und kapillarartigen Öffnungen können von den relativ kleineren Partikel vorteilhaft genutzt werden.

Ein weiterer wesentlicher Vorteil der Erfindung besteht darin, dass der Beschuss der Haut durch die beispielsweise bis zu 650 m/s sehr schnellen, größeren und tief in die Haut eindringenden Partikel mit einer Eindringzeit bei etwa nur 10 bis 50 Mikrosekunden erfolgt und folglich der Mensch bzw. das Tier dieses tiefe Eindringen nicht mehr als Schmerz verspürt, obwohl die Eindringfläche bzw. die beschossene Flüche sehr groß sein kann und sicher viele Schmerzrezeptoren beim Auftreffen getroffen bzw. gereizt werden.

Gegenüber dem Stand der Technik und zwar allen flüssigen nadelfreien Injektionsverfahren, kann eine Schmerzfreiheit nur erreicht werden, falls der Injektionskanal einen sehr kleinen Durchmesser von maximal 0,15 mm aufweist und folglich von den Schmerzrezeptoren der Haut nicht erfasst werden.

Im Rahmen dieser Erfindung bedeutet "Partikelauftragung (25)" solche Partikel, die zumindest einen Stoff oder Wirkstoff enthalten und beispielsweise mit Ölen u.a. an der äußeren (hautseitigen) Membran (15) fixiert sind oder durch Adhäsion anhaften, also auf der Membran beauftragt sind. Beispielsweise können die aufgebrachten Partikel Mittel enthalten, die ein Trocknen der Partikel auf der besagten Membran erlauben. Insbesondere sind zusätzliche Haftvermittler oder Additive geeignet und beizufügen, wie Öle oder andere vorzugsweise pharmazeutisch geeigneten Hilfs- und Zusatzstoffe.

Unter Partikelauftragung (25) werden ebenfalls Gel- und Flüssigkeitsteilchen verstanden, die eine Größe von 1 nm - 1.000 µm aufweisen können. Diese Gel- oder Flüssigkeitsteilchen können aus einem Gel- oder Flüssigkeitsfilm auf der Membran durch dessen stoßartiger Beschleunigung und Abbremsung bei der Ablösung des Gels bzw. der Flüssigkeit entstehen.

Der Begriff "Partikel enthaltend oder bestehend aus einem Stoff" umfasst sämtliche und beliebige Stoffe, Substanzen, Materialien oder Mittel.

Beispielsweise können die größeren Partikel eine Größe von 1, 10, 20, 40 - 1.000 µm aufweisen, während die kleineren Partikel 1-1.000 nm groß sind (z.B. so genannte Submikronteilchen), die auch z.B. bei 650 m/s Auftreffgeschwindigkeit niemals in die Haut eindringen können. Beispielsweise können die größeren Partikel als Öffner Glas-, Kunststoff oder Metallkügelchen, bzw. bei der Injektion in die Haut vornehmlich Zuckerkristalle sein, um diese Teilchen dann wieder abbauen zu können, während die kleineren Partikel einen Wirkstoff enthalten. Selbstverständlich ist der Fachmann in der Lage geeignete andere Relationen vom kleinen zu großen Partikel zu wählen.

Bereits bei einer Flugstrecke von mindestens 5 mm, insbesondere 5 bis 10 mm wird ein hinreichender Effekt erzielt, wie es die folgenden Beispiele und Figuren zeigen. Dies wird weiter unten unter der erfindungsgemäßen Beabstandung näher erläutert.

Wesentlich ist, dass eine hinreichende Impulsübertragung auf eine Membran (15), die auch als Doppelmembran bzw. Mehrschichtmembran ausgebildet sein kann, erfolgt, so dass die Partikel hinreichend beschleunigt werden und zwar vorzugsweise auf Geschwindigkeiten von 600 m/s und mehr.

Im Rahmen dieser Erfindung wird daher unter "Impuls(übertragung)" auch synonym eine Kraft-, Druckübertragung oder Beaufschlagung verstanden, welche jedenfalls ausreichend ist, die auf der hautseitigen Membran (15) aufgebrachte Partikelauftragung (25) auf die gewünschten hohen Geschwindigkeiten zu beschleunigen und abzulösen, wodurch sie zunächst frei in Richtung des Gewebes bzw. Haut (18) oder Körper fliegen, um dort einzuschlagen, die Gewebe- oder Körperbarriere zu durchbrechen und in die gewünschte Tiefe des Gewebes, insbesondere der Haut oder des Körpers einzudringen. Die fliegenden Partikel auf der Flugbahn können als Partikelstrahl beschrieben werden, wobei Agglutinationen von Partikel zu Partikel-Clustern erfolgen können.

Im Rahmen dieser Erfindung bedeutet "Haut" eine Gewebebarriere eines Menschen, Säugetier oder Tier. Die Haut übt als das größte Organ des Menschen zahlreiche lebenswichtige Funktionen aus, insbesondere die Epidermis, dient als Barriereorgan. Diese Barrierefunktion wird unter anderem von Hautlipiden aufrechterhalten. Diese epidermalen Lipide wie z.B. Glycosphingolipide, Ceramide, Sterine und Sterinester, Fettsäuren, Triglyceride, n-Alkane oder verschiedenen polaren Lipiden werden im Keratinisierungsprozess freigesetzt. Durch die erfindungsgemäße nadellose oder nadelfreie Applikation können infolge dessen die Stoffe in den Körper lokal eintreten und ggfs. in die Blutbahnen münden.

Die erforderliche Aktivierungsenergie zur Impuls(übertragung) kann daher mittels einer Aktivierungseinheit erfolgen, und zwar mittels Stoß oder vorzugsweise Explosion oder Detonation, insbesondere nicht abschließend einem Detonator (10), Anzünder, Zündstift oder Anzündstück und zwar über einen Auslösungsmechanismus mittels Reibung, Stoßen oder geeigneter Stromversorgung.

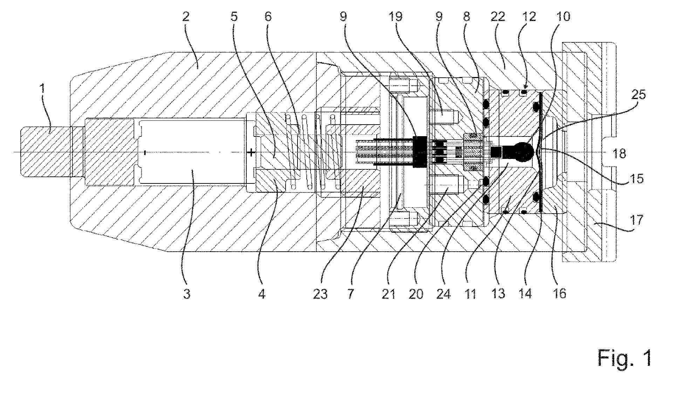

Eine geeignete Aktivierungseinheit ist beispielsweise in Figur 1 dargestellt. Mittels eines Auslösers oder Tasters (1) wird die Batterie (3) gegen eine Kontaktfeder (6) und gegen einen Kontaktstift (5) gedrückt, dadurch der Stromkreis über die Anschlüsse (9) und das EED (10) geschlossen und infolge die Zündung des EEDs (10) herbeigeführt. Alternative Auslösungsmechanismen sind dem Fachmann bekannt (Reibdraht, Schlagdraht, Knackfrosch auf stoßempfindliche Anzündmischung schlagend etc.).

In einer weiteren bevorzugten Ausführungsform ist die besagte Aktivierungseinheit (1, 2, 3, 4, 5, 6, 7, 8, 9, 10) in axialer Richtung zur Kammer (11) samt Verbrennungsraum (24) ausgerichtet und der Membran (15) gegenüberliegend.

Die erfindungsgemäße Membran (15) kann fixiert oder beweglich bzw. verschiebbar sein und ebenfalls als Doppelmembran (13, 15) bzw. Mehrschichtmembran ausgestaltet sein und folglich ist eine solche Membran erfindungsgemäß der Adressat einer solchen Impulsübertragung, so dass ein entsprechender Partikelstrahl (supra) entstehen kann.

In einer bevorzugten Ausführungsform besteht die erfindungsgemäße Membran (15) aus Metall oder einem Material entsprechender Härte und Duktilität, solche wie Stahl, Kunststoffen, besonders bevorzugt jedoch Titan oder Titanblech. Insbesondere Titan weist eine vorteilhafte hohe Ziehgrenze bei sehr guter Beschleunigbarkeit durch seine geringere spezifische Dichte gegenüber Stahl auf, wodurch bei relativ kleinen Drücken in der Brennkammer eine deutlich höhere Geschwindigkeit der Membranoberfläche bzw. der ausbeulenden Membran erreicht wird. Hier maßgeblich ist die Beschleunigungskennzahl BK=Fließgrenze sigma_{0,2} / Dichte ro.

Anstelle einer Einfachmembran kann ebenfalls eine Doppelmembran vorgesehen sein, d.h. beispielsweise statt einer 1 mm starken Einfachmembran werden zwei Membranen mit jeweils 0,5 mm übereinandergelegt, um eine Doppelmembran auszubilden. Ebenfalls andere Stärkeverhältnisse sind möglich, wie beispielsweise 0,6 mm Richtung Kammer und 0,4 mm Richtung Haut.

Eine Mehrschichtmembran liegt erfindungsgemäß vor, wenn mehr als 2 Schichten übereinandergelegt werden. So kann beispielsweise zwischen der Doppelmembran eine randnahe Dichtschicht oder eine Dämmschicht eingebracht sein, um die Geschwindigkeitsverteilung der ablösenden Teilchen zu verändern.

Im Fall einer fixierten Doppelmembran (13, 15) sind beide Membranen - die Doppelmembran - mit einer Stützscheibe (16), insbesondere an die Kammer (11), angebracht / fixiert und folglich im Randbereich nicht beweglich. Weiterhin ist bevorzugt, dass die nach innen gerichtete brennkammerseitige Membran (13) eine Dicke von 0,1 mm bis 0,6 mm aufweist, vorzugsweise 0,2 mm bis 0,6 mm, besonders bevorzugt 0,5 mm. Die nach außen gerichtete hautseitige Membran (15) weist vorzugsweise ebenfalls eine Dicke von 0,1 mm bis 0,6 mm auf, vorzugsweise 0,2 mm bis 0,6 mm besonders bevorzugt 0,5 mm. Die Membranen (13, 15) können sich ganz oder teilweise berühren.

In einer weiteren bevorzugten Ausführungsform weist jedoch die innere Membran (13) zur hautseitigen Membran (15) einen Abstand auf und zwar vorzugsweise von 0,2 mm bis 1,5 mm, besonders bevorzugt 1 mm. Der erforderliche Abstand kann mittels eines Abstandhalters (14) zwischen den beiden Membranen ausgelegt werden. Diese Maßnahme bewirkt eine effiziente Verhinderung des Platzens der hautseitigen Membran.

In einer weiteren besonders bevorzugten Ausführungsform ist die Membran (15) oder Doppelmembran (13, 15) zur Kammer (11) und in dessen Verbrennungsraum (24) hin ganz oder teilweise ausgewölbt, ganzflächig oder vorzugsweise im zentralen radialen Bereich um einen Kreismittelpunkt. Eine solche jeweils konkave Wölbung der jeweiligen Membranen aus Sicht der Brennkammer kann der Fachmann mittels bekannten Methoden erreichen. Die besagte erfindungsgemäße Wölbung der Membran (13) oder Doppelmembran (13, 15) erlaubt zudem eine vorteilhafte erhöhte Geschwindigkeit von 10 bis 15 % samt Fokussierung und Beschleunigung der aufgetragenen Substanzen (25) in Richtung Haut (18) - vermutlich durch Stoßwellenfokussierung.

In einer weiteren bevorzugten Ausführungsform weisen die beiden Membranen (13, 15) Stützscheiben (16) auf. Die Stützscheiben sind vorzugsweise aus Kunststoff, Messing etc., welche zur energetischen Aufnahme besonders geeignet ist. Darüber hinaus ist die Stützscheibe energetisch angepasst, vorzugsweise mit einer Verjüngung zur Haut (18) hin.

Gleichzeitig kann die Stützscheibe (16) an seiner Innenkontur mit einem weichen Kunststoff dünn überzogen sein, z.B. mit Polyethylen, um das Auslösegeräusch der Vorrichtung vorteilhaft deutlich zu reduzieren - es wird beispielsweise das knallartige Geräusch vermieden, welches entsteht, wenn eine Metallmembran auf eine metallene Stützscheibe aufschlägt.

Ein grundlegender und gattungsgemäßer Aufbau einer nadellosen Injektionsvorrichtung mit fixierter Membran ist z.B. in Figur 1 samt Legende gezeigt, wobei für die erfindungsgemäße Funktion der Injektionsvorrichtung das Gehäuse (22), sowie die Teile (7) bis (16) zu berücksichtigen sind. Die Teile (7) bis (16) können hierbei jederzeit in ein anders geformtes Gehäuse (22) eingebaut und ein Detonator, insbesondere EED (10), auch anders mit Energie versorgt werden, ohne die Funktion der Teile bzw. der Vorrichtung zu verändern. Besondere Ausführungsformen werden näher beschrieben.

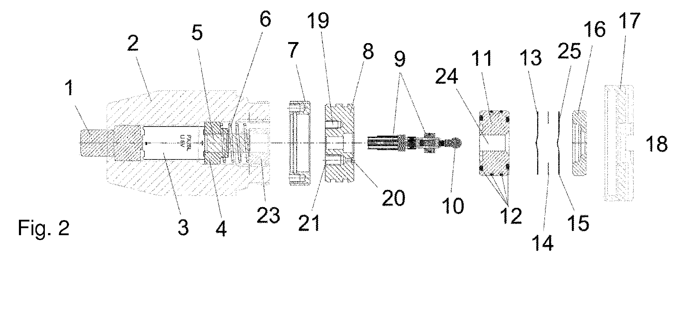

Die Geometrie der einzelnen Teile in Figur 1 können je nach Anforderung geändert oder teilweise integral zusammengefasst werden. Beispielsweise können Teile (7) bis (9) zu einem Teil integral zusammengefasst werden, oder Teil (7) kann entfallen, falls Teil (8) beispielsweise ein Außengewinde aufweist und in das Gehäuse (22) eingeschraubt wird-oder beispielsweise per Sprengring im Gehäuse (22) gehalten wird - oder ein Einlegeteil in der Spritzform des Gehäuses (22) ist (siehe ebenfalls Figur 2). Ebenfalls können die Teile (1), sowie die Teile (4) bis (6) und (23) durch die Verwendung eines herkömmlichen Tasters zur elektrischen Kontaktierung des Detonators (EED) (10) mit der Batterie (3) entfallen.

Weiterhin ist erfindungsgemäß bevorzugt, dass die nadelfreie Vorrichtung eine Kammer aufweist, die ein pyrotechnisches Material enthält, so dass mittels Explosion oder Detonation eine Impulsübertragung erfolgen kann.

Im Rahmen dieser Erfindung wird unter "pyrotechnisches Material" ein jedes Material verstanden, welches mit einer Aktivierungsenergie zur Explosion gebracht werden kann. Dies können z.B. feste oder gasförmige Stoffe, wie Azide, Tetrazen, Nitrozellulose, Pikrinsäure, etc. oder andere dem Fachmann bekannte pyrotechnische Materialien sein. Erfindungswesentlich ist, dass die Explosionsenergie eine hinreichende Impulsübertragung auf die erfindungsgemäße Membran (15) erlaubt, so dass die Partikelauftragung (25) auf hohe Geschwindigkeiten gebracht werden kann und letztlich die Partikel abgelöst werden.

Das pyrotechnische Material ist erfindungsgemäß insbesondere in einer nach außen vollständig geschlossenen Kammer (11) enthalten und zwar im Verbrennungsraum (24), vorzugsweise in Form eines Detonators (10), insbesondere in Form eines Minidetonators (EED) (10). Zusätzlich kann weiteres pyrotechnisches Material mit eingebracht sein.

In einer weiteren Ausführungsform kann der Verbrennungsraum (24) in der Kammer (11) vorzugsweise mit einer viskosen zähflüssigen Masse versehen sein, wie Fett, Öle und dergleichen, zwecks Verkleinerung des Leerraumes im Verbrennungsraum (24), wodurch eine Erhöhung des Druckanstieges bei kleiner Aufladung des Detonators (EED) (10) und zugleich eine optimale Ankopplung der vom Detonator ausgehenden Stoß- oder Druckwelle an die Membran bewirkt wird.

Die Kammer (11) weist vorzugsweise O-Ringe zur Abdichtung (12) auf. Weiterhin kann die Kammer (11) eine Entlüftung (20) samt zugehöriger Abdeckung (21) aufweisen.

In einer weiteren bevorzugten Ausführungsform enthält die Injektionsvorrichtung einen Aufsatz (17) und / oder Stützscheibe (16), so dass zwischen der Haut (18) und der Membran (15) ein definierter Abstand von beispielsweise ca. 5- 10 mm erreicht wird (supra), um die sich auswölbende Membran sicher von der Haut bzw. der Oberfläche des Körpers entfernt zu halten, als auch eine definierte Flugstrecke zu erhalten, wobei die Membran vorzugsweise parallel zur Haut beabstandet ist. Diese vorgenannte Beabstandung erlaubt einen hinreichenden Vorsprung der beschleunigten größeren Partikel gegenüber den kleineren Partikeln aus einer Partikelauftragung (25) an einer Membran (15).

Die vorgenannten Ausführungsformen erlauben daher vorteilhaft das Einbringen oder Penetrieren der Partikel und zwar der kleineren Partikel in die Haut oder einen Körper und zwar durch Öffnungen, die ein oder mehrere größere, schnellere Partikel vor Eintreffen der kleineren Partikel in der Haut oder im Körper nach dem Auftreffen verursacht haben. Weiterhin wird vorteilhaft durch die Ausgestaltung der Vorrichtung mit Membran bzw. des Applikationsgeräts mit Membran erreicht, dass die auf der Membran aufgebrachten Partikel eine optimierte Impulsübertragung bzw. Beschleunigung auf sehr hohe Geschwindigkeiten unter Ausbildung eines Partikelstrahls bis zu 800 m/s erfahren.

Weiterhin betrifft die Erfindung die Verwendung oder Anwendung von ein oder mehreren Stoffen, insbesondere Wirkstoffen, insbesondere eines Arzneimittels zur nadelfreien Applikation mittels der erfindungsgemäßen Injektionsvorrichtung nach einer der vorstehenden Ausführungsformen.

Weiterhin betrifft die Erfindung die Verwendung oder Anwendung von Stoffen, insbesondere Stoffen mit beliebiger Funktion und Eignung, solche wie Farb- oder Schmierstoffen oder Materialien mit anderen besonderen Eigenschaften zur nadellosen Einbringung in bzw. unter die Oberflächen oder Körper technischer Gegenstände mittels der erfindungsgemäßen Injektionsvorrichtung nach einer der vorstehenden Ausführungsformen.

Daher betrifft die Erfindung eine Injektionsvorrichtung nach einer der vorstehenden Ausführungsformen zur Verwendung eines Stoffes oder eines Wirkstoffes, insbesondere Arzneimittel, zur nadelfreien Applikation.

Die folgenden Beispiele und Figuren sollen die Erfindung näher erläutern, ohne jedoch diese zu beschränken.
Figur 1 zeigt einen Querschnitt der erfindungsgemäßen Injektionsvorrichtung mit fixierter Membran.
Figur 2 zeigt die Bestandteile zur Herstellung der erfindungsgemäßen Injektionsvorrichtung mit fixierter Membran deutlicher.

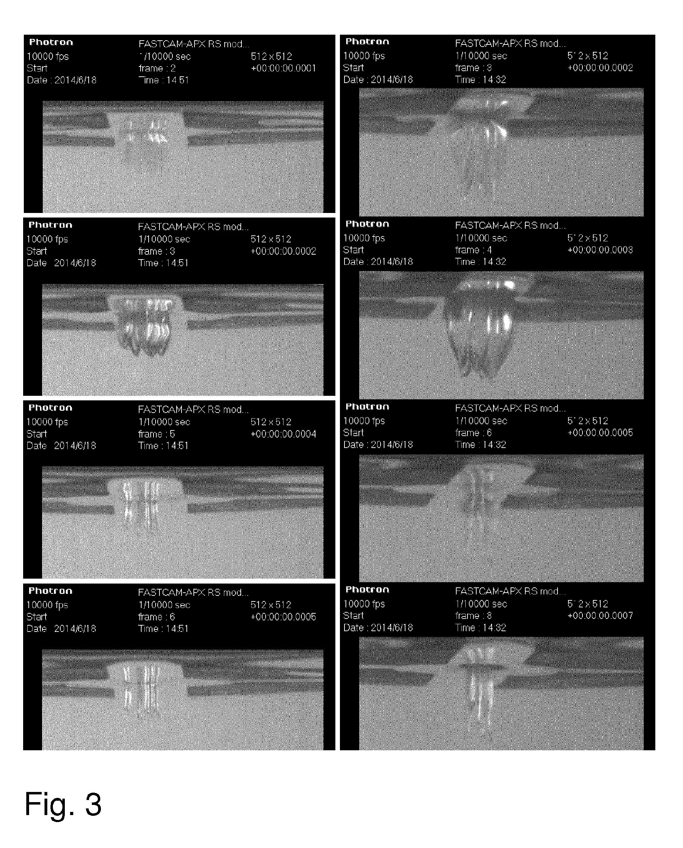

Figur 3: Bildsequenz, linke Spalte:
   Einschuss von ca. 10 Stück 250 µ großen Glaskugeln, die als Penetrationskörper verwendet werden, mit etwa 650 m/s in ein hautähnliches Gel. Man erkennt im ersten Bild oben bereits den Aufbau der Stoßkegel im Gel (schräger Verdichtungsstoß), die sich verstärken bzw.
   weiter aufbauen, um anschließend jeweils einen relativ dünnen, luftgefüllten Faden / Schlauch über dem jeweiligen Penetrationskörper übrig zu lassen (Bild unten).
Bildsequenz, rechte Spalte:
Einschuss von 5 Stück 500 µ großen Glaskugeln (Partikel), die als Penetrationskörper verwendet werden, mit etwa 650 m/s in ein hautähnliches Gel. Man erkennt im ersten Bild oben bereits den Aufbau der Stoßkegel im Gel (schräger Verdichtungsstoß), die sich dann verstärken, um anschließend einen relativ dünnen, luftgefüllten Faden / Schlauch über dem jeweiligen Penetrationskörper übrig zu lassen (Bild unten, die Fäden sind hier noch etwas aufgebläht).

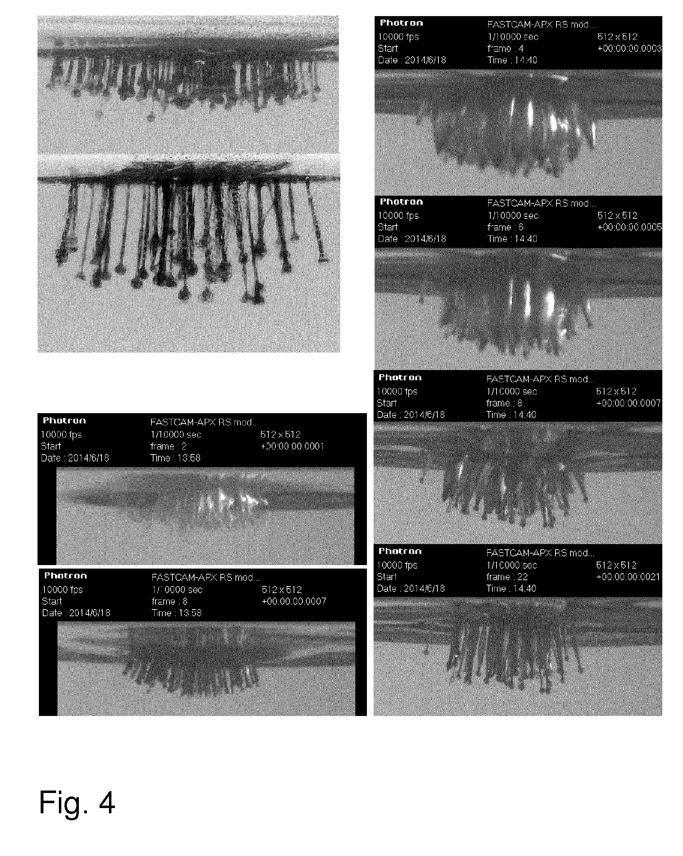

Figur 4: Bildsequenz oben links = 2 Bilder, ca. 50 Stück Glaskugeln (Partikel) mit einem Durchmesser von 500 µm werden in künstliche Haut eingeschossen, auf der Membran bestäubt mit 20 bis 80 µm großen Zuckerpartikeln, die schwarz eingefärbt sind.

Bildsequenz unten links=2 Bilder, ca. 100 Stück 250 µm große Glaskugeln, auf der Membran bestäubt mit 20 bis 80 µm großen Zuckerpartikeln, die schwarz eingefärbt sind.

Bildsequenz rechts=4 Bilder dto. für 500µm große Glaskugeln / Penetrationskörper.

Jede der obigen 3 Bildsequenzen zeigt die Situation nach dem Auslösen des nadelfreien Injektors bzw. des pyrotechnischen Membrangerätes, wenn zu den Penetrationskörpern auf der hautseitigen Membran auch zugleich kleine Teilchen aufgebracht / aufgestäubt werden. Zuerst dringen die Penetrationskörper hier mit etwa 650 m/s ein, dann baut sich ein Stoßkegel über jedem Penetrationskörper auf, die eine große innere Oberfläche für die nachfolgend einfliegenden, mit etwa 620 m/s etwas langsameren Teilchen ausbilden. Kurz danach "klatschen" die Stoßkegel wieder zusammen (die Geschwindigkeit der Penetrationskörper ist inzwischen unter die Schallgeschwindigkeit des Stoffes des penetrierten Körpers / der Haut / des Gels bzw. bis auf 0 m/s gesunken) und kompaktieren gleichsam die vorher eingebrachten Teilchen, die hiermit quasi gebunkert werden.

Wenn für medizinische Zwecke beispielsweise Zuckerteilchen als Penetrationskörper verwendet werden, werden diese nach Ihrer Aufgabe vom Körper / der Haut / dem Gewebe abgebaut, es verbleiben hiernach die in den von ihnen geschaffenen "Schläuchen" kompaktierten Teilchen.

### Legende

- 1: Auslöser, Taster
- 2: Auslösergehäuse
- 3: Batterie
- 4: Federkappe
- 5: Kontaktstift
- 6: Kontaktfeder
- 7: Nutmutter
- 8: Halter für Glasdurchführung
- 9: Glasdurchführung mit Anschlüsse
- 10: Minidetonator oder Anzündstück, EED
- 11: Brennkammer oder Kammer
- 12: O-Ring-Abdichtung der Brennkammer (kann bei einigen Ausführungsformen der Vorrichtung auch entfallen)
- 13: Innere Membran
- 14: Abstandshalter, übernimmt auch Abdichtfunktionen
- 15: Hautseitige Membran, fixiert zu Membran (13) und Abstandshalter (14)
- 16: Stützscheibe
- 17: Aufsatz
- 18: Haut oder die zu penetrierende Oberfläche (liegt auf 17 an, nicht gezeichnet)
- 19: Gewinde für Masseanschlussschraube (kann bei einigen Ausführungsformen der Vorrichtung auch entfallen)
- 20: Brennkammerentlüftung
- 21: Abdeckung, Entlüftung
- 22: Gehäuse für die Teile 7-21
- 23: Führung für Kontaktstift
- 24: Verbrennungsraum
- 25: Partikelauftragung

## Patentansprüche

1. Nadellose Injektionsvorrichtung umfassend eine hautseitige Membran (15), wobei die Membran (15) mit einer Partikelauftragung (25) versehen ist, wobei ein oder mehrere oder mindestens ein Teil der Partikel größer ist/sind als die restlichen Partikel.

2. Nadellose Injektionsvorrichtung nach Anspruch 1, wobei ein oder mehrere oder mindestens ein Teil der Partikel 2-fach, 5-fach, 10-fach oder 20-fach größer ist/sind als die restlichen Partikel.

3. Nadellose Injektionsvorrichtung nach Anspruch 1 oder 2, wobei die größeren Partikel am Gesamtanteil der Partikelauftragung (25) 1%, 5%, 10 % und mehr beträgt.

4. Nadellose Injektionsvorrichtung nach einem der vorstehenden Ansprüche, wobei zumindest die größeren Partikel aus einem pharmazeutisch verträglichen Material bestehen.

5. Nadellose Injektionsvorrichtung nach einem der vorstehenden Ansprüche, wobei zumindest die kleineren Partikel aus einem Stoff, insbesondere Wirkstoff bestehen oder einen Stoff, insbesondere Wirkstoff enthalten.

6. Nadellose Injektionsvorrichtung nach einem der vorstehenden Ansprüche, wobei die hautseitige Membran (15) eine Dicke von 0,1 mm bis 0,6 mm aufweist.

7. Nadellose Injektionsvorrichtung nach einem der vorstehenden Ansprüche, enthaltend einen Aufsatz (17) und / oder Stützscheibe (16), **dadurch gekennzeichnet, dass** zwischen der Haut (18) und der Membran (15) ein Abstand von mindestens 5 mm erreicht wird.

8. Nadellose Injektionsvorrichtung nach einem der vorstehenden Ansprüche, wobei die Membran als Mehrschicht oder Doppelmembran, bestehend aus einer inneren Membran (13) und einer hautseitigen Membran, (15) ausgebildet ist.

9. Nadellose Injektionsvorrichtung nach einem der vorstehenden Ansprüche, wobei mindestens eine Membran (13) oder (15) gewölbt ist.

10. Nadellose Injektionsvorrichtung nach einem der vorstehenden Ansprüche, wobei eine Aktivierungseinheit (1, 2, 3, 4, 5, 6, 7, 8, 9, 10) in axialer Richtung zur Membran (15) vorhanden ist.

11. Nadellose Injektionsvorrichtung nach einem der vorstehenden Ansprüche zur Verwendung eines Stoffes oder eines Wirkstoffes oder Arzneimittels zur nadelfreien Applikation.
